# EUROPÄISCHE PATENTSCHRIFT

(11) **EP 2 934 462 B1**
(45) Veröffentlichungstag und Bekanntmachung des Hinweises auf die Patenterteilung: **29.11.2017**
(21) Anmeldenummer: 13802947.5
(22) Anmeldetag: 09.12.2013
(51) Int. Cl.: A61K 8/37, A61K 8/46, A61Q 5/10, A61K 8/81, A61K 8/92, A61K 8/22

(54) **ZWEIPHASEN-ENTWICKLER FÜR OXIDATIVE FARBVERÄNDERUNGSMITTEL**
TWO-PHASE DEVELOPER FOR OXIDATIVE COLOR CHANGING AGENTS
RÉVÉLATEUR DIPHASIQUE POUR DES PRODUITS MODIFICATEURS DE COULEUR PAR OXYDATION

(30) Priorität: 18.12.2012 DE 102012223564
(43) Veröffentlichungstag der Anmeldung: 28.10.2015
(73) Patentinhaber: Henkel AG & Co. KGaA, 40589 Düsseldorf (DE)
(72) Erfinder: BABIEL, Sabine, 47447 Moers (DE); REICHERT, Anja, 40629 Düsseldorf (DE); BRAKE, Carsten, 45131 Essen (DE); GRUNWALD, Mechtild, 40764 Langenfeld (DE); AUGUSTIN-CASTRO, Barbara, NL-6291 Vaals (NL)
(86) Internationale Anmeldenummer: PCT/EP2013/075955
(87) Internationale Veröffentlichungsnummer: WO 2014/095458

(56) Entgegenhaltungen:
- EP-A1- 2 198 839
- EP-A2- 2 345 400
- WO-A2-2010/133575
- WO-A2-2011/082910
- DE-A1-102010 003 264

## Beschreibung

Gegenstand der vorliegenden Anmeldung ist ein kosmetisches Mittel für die Behandlung keratinischer Fasern, welches sich durch zwei voneinander getrennte Phasen auszeichnet, wobei eine der Phasen eine wässrige Phase und die andere Phase eine hydrophobe Ölphase darstellt. Diese Mittel enthalten mindestens ein chemisches Oxidationsmittel sowie eine Mischung aus Isopropylpalmitat und einem weiteren flüssigen, verzweigten Carbonsäureester. In ihrer Eigenschaft als oxidierende Zubereitungen werden diese Mittel als Entwicklerzubereitung für Oxidationsfärbungen oder Aufhellmittel eingesetzt. Ein weiterer Gegenstand der vorliegenden Erfindung ist daher ein Verfahren zur Farbveränderung keratinischer Fasern, bei dem die erfindungsgemäßen Mittel auf keratinische Fasern aufgebracht werden.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, die als färbende Komponente sogenannte direktziehende Farbstoffe ("Direktzieher") enthalten. Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Sollen Substrate aufgehellt oder gar gebleicht werden, werden die das Substrat färbenden Farbstoffe meist oxidativ unter Einsatz von entsprechenden Oxidationsmitteln, wie Wasserstoffperoxid, entfärbt.

Um eine optimale Färbeleistung zu entfalten, benötigen oxidative Färbemittel in der Regel einen alkalischen pH-Wert zur Ausfärbung, insbesondere zwischen pH 9,0 und pH 10,5. Darüber hinaus beträgt die Anwendungsdauer für ansprechende Färbeergebnisse üblicherweise zwischen 10 und 45 min. Es ist daher notwendig, dass das anwendungsbereite Färbemittel so formuliert und konfektioniert ist, dass das Färbemittel sich einerseits gut auf den zu färbenden keratinischen Fasern verteilen lässt, andererseits jedoch in den zu färbenden Fasern während der Anwendungszeit verbleibt. Dazu ist es vorteilhaft, wenn das Färbemittel über eine bestimmte Viskosität verfügt, die zwar das Auftragen des Mittels ermöglicht, jedoch das Mittel auch am Ort der Anwendung verbleiben lässt. Diese Viskosität kann durch polymere Verdickungsmittel im anwendungsbereiten Färbemittel eingestellt werden, wobei dieses Verdickungsmittel sowohl in der Farbveränderungszubereitung oder der Oxidationsmittelzubereitung enthalten sein kann.

Um eine gute Mischung von Farbveränderungszubereitung und Oxidationsmittelzubereitung zu ermöglichen, ist es vorteilhaft, wenn die Farbveränderungszubereitung und Oxidationsmittelzubereitung über eine gute Fließfähigkeit verfügen.

Daneben sollen die Oxidationsmittelzubereitungen auch über pflegende Eigenschaften verfügen und diese durch ein geeignetes optisches, kosmetisches Erscheinungsbild der Haare verdeutlichen. Dazu sind insbesondere zweiphasige Oxidationsmittelzubereitungen geeignet, die neben einer wässrigen, oxidationsmittelhaltigen Phase eine weitere pflegende, hydrophobe Phase enthalten.

Zweiphasige Oxidationsmittelzubereitungen mit pflegenden Eigenschaften sind aus DE102010003264 A1 bekannt. Darin werden zwei Phasen beschrieben, die in Kontakt miteinander verpackt werden, wobei eine Phase eine wässrige Oxidationsmittelphase und eine weitere Phase eine hydrophobe Ölphase darstellt.

Es wurde gefunden, dass die Lagerstabilität der Zusammensetzungen gemäß DE102010003264 A1 nicht immer zufrieden stellend ist. So konnte mitunter festgestellt werden, dass sich die Fließfähigkeit der hydrophoben Ölphase bei Temperaturschwankungen - insbesondere im Bereich kälterer Temperaturen - verändern kann, was die gute Vermischbarkeit der Farbveränderungszubereitung und der Oxidationsmittelzubereitung beeinträchtigt.

Aufgabe der vorliegenden Erfindung ist es daher, ein kosmetisches Mittel zur Verfügung zu stellen, durch welches die oben genannten Nachteile herabgesenkt werden. Insbesondere ist es die Aufgabe der vorliegenden Erfindung, eine zweiphasige Oxidationszubereitung für oxidative Farbveränderungsmittel von keratinischen Fasern zur Verfügung zu stellen, deren wässrige Phase und hydrophobe Ölphase sich auch bei längerer Lagerung und/oder bei Temperaturschwankungen (insbesondere bei Temperaturen unterhalb von 15°C) problemlos miteinander vermischen lassen. Insbesondere soll die Fließfähigkeit der hydrophoben Phase auch bei der Lagerung bei tieferen Temperaturen nicht verschlechtert werden.

Eine weitere Aufgabe der Erfindung ist die Verbesserung der Pflegeeigenschaften zweiphasiger Oxidationszubereitungen. Unter verbesserten Pflegeeigenschaften sind beispielsweise eine verbesserte Nass- und Trockenkämmbarkeit und/oder ein höherer Glanz der behandelten Haare und/oder die Erzielung einheitlicherer, gleichmäßigerer Färbeergebnisse zu verstehen.

In nicht vorhersehbarer Weise konnte nun gefunden werden, dass diese Aufgaben durch spezielle zweiphasige, oxidative Zubereitungen, die neben einem Oxidationsmittel eine Mischung aus Isopropylpalmitat und mindestens einem weiteren, flüssigen hydrophoben Öl enthalten, in besonderer Weise gelöst werden.

Ein erster Gegenstand der vorliegenden Erfindung ist daher ein kosmetisches Mittel für die Behandlung keratinischer Fasern, das mindestens zwei nebeneinander vorliegende, aber durch eine Phasengrenze voneinander getrennte Phasen umfasst, wobei die erste Phase (I) eine wässrige Phase darstellt, die mindestens ein chemisches Oxidationsmittel enthält, und wobei die zweite Phase (II) eine hydrophobe Ölphase darstellt, die eine Mischung aus Isopropylpalmitat (II-1) und mindestens einem weiteren flüssigen, verzweigten Carbonsäureester (II-2) ausgewählt aus 2-Ethylhexylpalmitat, Hexyldecylpalmitat, Hexyldecylstearat, Hexyldecyllaurat, Isodecylneopentanoat, Isononylisononanoat, 2-Ethylhexylstearat, Isooctylstearat, Isononylstearat, Isocetylstearat, Isotridecylisononanoat, Propylheptylcaprylat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat und/oder Diisotridecylacetat enthält.

Unter keratinhaltigen bzw. keratinischen Fasern werden erfindungsgemäß Pelze, Wolle, Federn und insbesondere menschliche Haare verstanden. Obwohl die erfindungsgemäße Verwendung in erster Linie zum Färben und/oder Aufhellen von keratinhaltigen Fasern geeignet sind, steht prinzipiell einer Verwendung auch auf anderen Gebieten nichts entgegen.

Entscheidendes Merkmal der erfindungsgemäßen Zubereitung ist dabei die Zweiphasigkeit, wobei die beiden Phasen nicht miteinander mischbar sind und wobei die beiden Phasen in zwei Schichten übereinander mit unmittelbarem Kontakt über eine gemeinsame Grenzfläche zueinander vorliegen.

In der erfindungsgemäßen Zubereitung liegt die Phase (I) bevorzugt zu mindestens gleichen Gewichtsanteil wie die Phase (II) vor. Bevorzugt liegt Phase (I) im Überschuss vor. Bevorzugt besitzt das Gewichtsverhältnis von Phase (I) zu Phase (II) einen Wert von 99 zu 1 bis 50 zu 50, bevorzugt von 98 zu 2 bis 70 zu 30, besonders bevorzugt von 95 zu 5 bis 80 zu 20.

Ein zwingendes Merkmal der vorliegenden Erfindung ist die Phase (I), die wässrig ist. Darunter wird verstanden, dass die Phase (I) eine wasserhaltige Zusammensetzung ist, die 3 bis 70 Gew.-% eines C₁-C₄-Alkohols, bezogen auf das Gesamtgewicht der Anwendungsmischung, enthalten kann. Bevorzugte C₁-C₄-Alkohole sind insbesondere Ethanol bzw. Isopropanol.

In einer bevorzugten Ausführungsform enthält das kosmetischde Mittel mindestens 30 Gew.-%, insbesondere mindestens 50 Gew.-% Wasser, bezogen auf das Gesamtgewicht des Mittels.

Weiterhin enthält die erste Phase (I) mindestens ein chemisches Oxidationsmittel. Der Begriff "chemisches Oxidationsmittel" soll dabei verdeutlichen, dass es sich dabei um ein extra zugesetztes Oxidationsmittel handelt und nicht etwa um ein in der Umgebung vorliegendes Oxidationsmittel, wie beispielsweise Luftsauerstoff. Als erfindungsgemäßes Oxidationsmittel wird bevorzugt Wasserstoffperoxid verwendet. Wasserstoffperoxid wird dabei entweder als vorzugsweise wässrige Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinon·n H₂O₂ (n ist eine positive ganze Zahl größer 0), Harnstoffperoxid und Melaminperoxid, eingesetzt. Erfindungsgemäß bevorzugte wässrige Phasen (I) enthalten wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt. Vorzugsweise werden 3 Gew.-%ige bis 12 Gew.-%ige Lösungen von Wasserstoffperoxid in Wasser verwendet.

Eine Ausführungsform des ersten Erfindungsgegenstands ist daher dadurch gekennzeichnet, dass das chemische Oxidationsmittel der Phase (I) ausgewählt ist aus Wasserstoffperoxid und/oder einem seiner festen Anlagerungsprodukte an anorganische und/oder organische Verbindungen.

Die erfindungsgemäßen Zubereitungen enthalten mit besonderem Vorzug Wasserstoffperoxid. Hier sind erfindungsgemäße Mittel zur Farbveränderung keratinischer Fasern besonders bevorzugt, die 0,5 bis 18 Gew.-%, vorzugsweise 1 bis 15 Gew.-%, besonders bevorzugt 2,5 bis 12 Gew.-% und insbesondere 3 bis 9 Gew.-% Wasserstoffperoxid (berechnet als 100%iges H₂O₂) enthalten.

Gemäß der vorliegenden Erfindung ist die zweite Phase (II) hydrophober Natur. Die erfindungsgemäße hydrophobe Phase (II) ist mit der wässrigen Phase (I), enthaltend das Oxidationsmittel, nicht mischbar. Hydrophobe Phasen, auch lipophile Phasen genannt, enthalten Fettkörper, die üblicherweise unpolare organische Verbindungen wie Kohlenwasserstoffverbindungen, langkettige Triglyceride, Siliconöle, Ester oder Ether sowie perhalogenierte Verbindungen enthalten.

Die hydrophobe Ölphase (II) der vorliegenden Erfindung zeichnet sich dadurch aus, dass sie eine Mischung aus Isopropylpalmitat (II-1) und mindestens einem weiteren, flüssigen, verzeigten Carbonsäureester (II-2) enthält. Der Begriff "flüssig" bezeichnet dabei bei Raumtemperatur und unter Normaldruck flüssige Carbonsäureester. Erfindungsgemäß geeignete Carbonsäureester sind solche, die keine oder nur eine sehr geringe Wasserlöslichkeit besitzen, d. h. eine Wasserlöslichkeit von unter 1 g pro 1 L Wasser bei Normalbedingungen.

Es wurde gefunden, dass die Kältestabilität der erfindungsgemäßen Mittel besonders ausgeprägt ist, wenn das Isopropylpalmitat (II-1) zu der Summe aller weiterer Carbonsäureester (II-2) in Phase (II) in einem bestimmten Gewichtsverhältnis vorliegt.

In einer weiteren bevorzugten Ausführungsform beträgt das Gewichtsverhältnis des Isopropylpalmitats (II-1) zu der Summe aller weiterer Carbonsäureester (II-2) in Phase (II) daher 20 : 80 bis 80 : 20, bevorzugt 30 : 70 bis 70 : 30, mehr bevorzugt 40 : 60 bis 60 : 40 und insbesondere bevorzugt 45 : 55 bis 55 : 45.

Unter geeigneten "flüssigen, verzweigten Carbonsäureestern" werden bevorzugt Ester geradkettiger oder verzweigter C₂-C₃₀-Carbonsäuren mit verzweigten C₃-C₃₀-Alkoholen verstanden, wobei unter dem Begriff "Carbonsäuren" sowohl Mono- als auch Dicarbonsäuren zu verstehen sind. Bevorzugt sind die Monocarbonsäureester.

Beispiele für erfindungsgemäß geeignete C₃-C₃₀-Alkohole sind Isopropanol, Isobutanol, 2-Methyl-2-butanol, 2-Methyl-3-butanol, 2-Methyl-2-pentanol, 2-Methyl-3-pentanol, 2-Methyl-4-pentanol, 2-Ethylhexanol, 2-Ethylheptanol, Isooctanol, Isononanol, Isodecylalkohol, Isotridecanol, Hexyldecylalkohol, Octyldodecanol und/oder Triisodecanol.

Beispiele für erfindungsgemäß geeignete Carbonsäuren sind Essigsäure, Propansäure, Octansäure, Neooctansäure, Neopentansäure, Nonansäure, Isononansäure, Decylsäure, Neodecansäure, Laurinsäure, Isolaurinsäure, Myristinsäure, Isomyristinsäure, Kokossäure, Palmitinsäure, Isopalmitinsäure, Stearinsäure, Isostearinsäure und/oder Oleinsäure. Flüssige, verzweigte Carbonsäureester, die sich für eine Vermischung mit Isopropylpalmitat in Phase (II) eignen, sind ausgewählt aus 2-Ethylhexylpalmitat, Hexyldecylpalmitat, Hexyldecylstearat, Hexyldecyllaurat, Isodecylneopentanoat, Isononylisononanoat, 2-Ethylhexylstearat, Isopropyloleat, Isooctylstearat, Isononylstearat, Isocetylstearat, Isononylisononanoat, Isotridecylisononanoat, Cetearylisononanoat, Propylheptylcaprylat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat und/oder Diisotridecylacetat.

Besonders lager- und kältestabile erfindungsgemäße Mittel können erhalten werden, wenn als weiterer flüssiger verzweigter Carbonsäureester mindestens ein Ester verwendet wird, der einen Trübungspunkt < 7°C aufweist.

In einer weiteren bevorzugten Ausführungsform wird daher das Isopropylpalmitat der Phase (II) mit mindestens einem der zuvor genannten Ester vermischt, der bevorzugt einen Trübungspunkt < 7°C, mehr bevorzugt < 5°C und insbesondere < 3°C aufweist.

In einer ersten besonders bevorzugten Ausführungsform weist die Phase (II) eine Mischung von Isopropylpalmitat und 2-Ethylhexylpalmitat (Octyl Palmitate) auf.

In einer zweiten besonders bevorzugten Ausführungsform weist die Phase (II) eine Mischung von Isopropylpalmitat und Hexyldecylpalmitat auf.

In einer dritten besonders bevorzugten Ausführungsform weist die Phase (II) eine Mischung von Isopropylpalmitat und Hexyldecylstearat auf.

Aus Stabilitätsgründen ist es essentiell, dass die beiden Phasen (I) und (II) klar und scharf voneinander getrennt vorliegen und dass keine Zwischenphasen oder Mischphasen im Phasengrenzbereich auftreten. Auch sollen Eintrübungen der Phasen durch Schlierenbildungen oder partiellen Emulsionsbereichen in den Phasen vermieden werden. Es ist daher von besonderem Vorteil, wenn insbesondere die hydrophobe Ölphase klar und transparent vorliegt. Unter "klar" und "transparent" ist hierbei eine Transmission von wenigstens 60%, bevorzugt wenigstens 80% zu verstehen. Diese Transmission wird mit UV/VIS-Spectrometrie gemessen. Die Transmission kann dabei bei unterschiedlichen Wellenlängen gemessen werden, bevorzugt bei Raumtemperatur, bei einer Wellenlänge von λ = 600 nm und einer Küvettenlänge von 10 mm.

Bevorzugt enthält das Mittel des ersten Erfindungsgegenstands 3 bis 30 Gew.-%, mehr bevorzugt 4 bis 25 Gew.-%, besonders bevorzugt 5 bis 20 Gew.-% und insbesondere 7,5 bis 15 Gew.-% der hydrophoben Ölphase (II).

Diese kann neben der Mischung aus Isopropylpalmitat und dem mindestens einen flüssigen, verzweigten Carbonsäureester gegebenenfalls weitere Ölkomponenten wie beispielsweise Kohlenwasserstoffverbindungen, langkettige Triglyceride, Siliconöle, Ester oder Ether sowie perhalogenierte Verbindungen enthalten.

Ein Ziel der vorliegenden Erfindung war es zweiphasige Mittel herzustellen, wobei beide Phasen eine ausreichende Fließfähigkeit aufweisen sollten, damit unmittelbar vor der Anwendung eine gute Durchmischung der Phasen erreicht werden kann. Optimalerweise sollte sich die Viskosität des Mittels nach der Durchmischung der beiden Phasen jedoch erhöhen, damit das Mittel einfacher auf die Haarfasern aufgetragen werden kann, und dort für die Dauer der Anwendung verbleibt ohne auszulaufen.

Für diesen Zweck hat es sich als vorteilhaft erwiesen, der wässrigen Phase neben dem chemischen Oxidationsmittel mindestens ein geeignetes polymeres Verdickungsmittel hinzuzufügen.

Besonders bevorzugte erfindungsgemäße Mittel sind in einer weiteren Ausführungsform daher dadurch gekennzeichnet, dass sie in der wässrigen Phase (I) weiterhin mindestens ein anionisches, polymeres Verdickungsmittel enthalten. Bevorzugte anionische polymere Verdickungsmittel sind ausgewählt aus vernetzten oder unvernetzten Copolymeren, die mindestens zwei unterschiedliche Monomere aus der Gruppe der Acrylsäure, Methacrylsäure, den C₁-C₆-Alkylestern der Acrylsäure und/oder den C₁-C₆-Alkylestern der Methacrylsäure enthalten.

Besonders bevorzugte anionische Copolymere sind Copolymere aus Acrylsäure, Methacrylsäure oder deren C₁-C₆-Alkylestern, die unter der INCI-Bezeichnung Acrylates Copolymer vertrieben werden. Insbesondere bevorzugt ist die Kombination aus Methacrylsäure und Ethylacrylat sowie gegebenenfalls vernetzenden, multifunktionalen Monomeren. Ein bevorzugtes Handelsprodukt dafür ist beispielsweise Aculyn® 33 bzw. 33A, das von der Firma Rohm & Haas angeboten wird.

Die anionischen polymeren Verdickungsmittel können bevorzugt in einer Gesamtmenge von 0,1 bis 15 Gew.-%, mehr bevorzugt von 1 bis 10 Gew.-% und insbesondere von 1,5 bis 7,5 Gew.-% eingesetzt werden, wobei sich die Mengen auf das Gesamtgewicht des erfindungsgemäßen Mittels beziehen.

Um die Trennung von hydrophiler Phase (I) und hydrophober Phase (II) zu verbessern und eine klare, hydrophobe Phase (II) zu erhalten, muss die Neigung des Mittels zur Bildung einer stabilen Emulsion reduziert sein. Daher ist es erfindungsgemäß bevorzugt, wenn das Mittel nur einen geringen Anteil an grenzflächenaktiven Substanzen enthält. Als grenzflächenaktive Substanzen im Sinne der Erfindung gelten dabei Emulgatoren und Tenside. Grenzflächenaktive Substanzen zeichnen sich durch hydrophobe und hydrophile Strukturmerkmale aus und ermöglichen so eine Durchmischung der Phasen unter Ausbildung von Micellen und stabilen Emulsionen. Da die vorliegende Erfindung explizit keine Emulsionen umfasst, sondern vielmehr zwei getrennt voneinander vorliegende Phasen enthält, hat es sich als erfindungsgemäß besonders vorteilhaft herausgestellt, dass das Mittel nichtionische, anionische, zwitterionische und/oder amphotere Tenside und/oder Emulgatoren in einem Gesamtgewicht von weniger als 5 Gew.-%, mehr bevorzugt von weniger als 3 Gew.-%, weiter bevorzugt von weniger als 1 Gew.-%, außerordentlich bevorzugt in einem Gesamtgewicht von 0,05 bis 0,5 Gew.-%, besonders bevorzugt 0,1 - 0,3 Gew.-%, jeweils bezogen auf das Gesamtgewicht des erfindungsgemäßen Mittels, enthält.

Anionische Tenside im Sinne der Erfindung sind alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen); Ethercarbonsäuren, insbesondere der Formel RO(CH₂CH₂O)ₓCH₂COOH, in der R eine lineare Alkylgruppe mit 8 bis 30 C-Atomen und x = 0 oder 1 bis 16 ist; Acylsarcoside; Acyltauride; Acylisethionate; Sulfobernsteinsäuremono- und -dialkylester sowie Sulfobernsteinsäuremono-alkylpolyoxyethylester; lineare Alkansulfonate; lineare α-Olefinsulfonate; Sulfonate ungesättigter Fettsäuren; α-Sulfofettsäuremethylester von Fettsäuren; Alkylsulfate und Alkylethersulfate, insbesondere der Formel RO(CH₂CH₂O)ₓSO₃H, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate der Formel RO(C₂H₄O)ₓP(=O)(OH)(OR'), worin R für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht; sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht; sowie Monoglyceridsulfate und Monoglyceridethersulfate.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Beispiele solcher zwitterionischen Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Übliche amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Beispielhafte amphotere Tenside sind N-Kokosalkylaminopropionat, Kokosacylaminoethylaminopropionat und C₁₂-C₁₈-Acylsarcosin.

Nichtionische Tenside und Emulgatoren enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; mit einem Methyl- oder C₂-C₆-Alkylrest endgruppenverschlossene Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol LS, Dehydol LT (BASF) erhältlichen Typen; Polyglycerinester und alkoxylierte Polyglycerinester, wie beispielsweise Poly(3)glycerindiisostearat (Handelsprodukt: Lameform TGI (BASF)) und Poly(2)glycerinpolyhydroxy-stearat (Handelsprodukt: Dehymuls PGPH (BASF)); Polyolfettsäureester, wie beispielsweise das Handelsprodukt Hydagen HSP (BASF) oder Sovermol-Typen (BASF); höher alkoxylierte, propoxylierte und insbesondere ethoxylierte, Mono-, Di- und Triglyceride mit Alkoxylierungsgrad von größer als 5, wie beispielsweise Glycerinmonolaurat + 20 Ethylenoxid und Glycerinmonostearat + 20 Ethylenoxid; Aminoxide; Hydroxymischether; Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO); Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate mit 6 bis 21, insbesondere 6 bis 15 Kohlenstoffatomen in der Alkylkette und 5 bis 30 Ethylenoxid- und/oder Propylenoxid-Einheiten; Alkylpolyglykoside entsprechend der allgemeinen Formel RO-(Z)ₓ, wobei R für Alkyl, Z für Zucker sowie x für die Anzahl der Zuckereinheiten steht.

Zu den nichtionischen Emulgatoren im Sinne der Erfindung zählen weiterhin die Polymerisationsprodukte aus Ethylenoxid und Propylenoxid an gesättigte oder ungesättigte Fettalkohole; Alkylester von gesättigten oder ungesättigten Fettsäuren oder Alkylphenole und deren Alkoxylate; insbesondere Ethylenglykolether von Fettalkoholen; gemischte Ethylen- und Propylenglykolether mit Fettalkoholen; Fettsäureester an Sorbitan sowie Polyethylenglykol; Ester von nicht hydroxylierten C₆-C₃₀-Alkylmono-carbonsäuren mit Polyethylenglykol; und Anlagerungsprodukte von Alkylphenolen an Ethylen- und/oder Propylenoxid.

Weiterhin kann es zur Trennung der hydrophilen und hydrophoben Phase im erfindungsgemäßen Mittel vorteilhaft sein, zusätzlich dem Mittel Elektrolyte zuzugeben. Unter Elektrolyten werden üblicherweise geladene, ionische anorganische und organische Verbindungen verstanden, welche über keinen oder nur einen nur sehr gering ausgeprägten hydrophoben Anteil enthalten. Bevorzugte Elektrolyte sind gut wasserlösliche Salze, insbesondere Alkalimetall- und Erdalkalimetallsalze von Mineralsäuren und organischen Säuren. Beispiele hierfür sind Natriumchlorid, Natriumsulfat, Natriumhydrogensulfat, Natriumcarbonat, Natriumhydrogencarbonat, Natriumcitrat, Magnesiumchlorid, Magnesiumsulfat, Magnesiumcarbonat und Magnesiumhydrogencarbonat.

Das erfindungsgemäße Mittel zeichnet sich dadurch aus, dass sich öllösliche Inhaltsstoffe überwiegend in der hydrophoben Phase (II) anreichern und daher nicht in unmittelbaren Kontakt mit der Oxidationsmittel-haltigen Phase (I) kommen. Dies ist besonders vorteilhaft, um oxidativ wenig stabile Pflegestoffe im Mittel zu stabilisieren. Solche bevorzugte Pflegestoffe sind daher öllösliche Pflegestoffe, öllösliche Vitamine und Triglyceride, insbesondere pflanzliche und solche, die eine oder mehrere ungesättigte Kohlenstoff-Kohlenstoff-Bindungen enthalten. Um die Zweiphasigkeit optisch zu verdeutlichen, kann es ebenso sinnvoll sein, wenn die hydrophobe Phase (II) öllösliche Farbstoffe enthält.

Eine besondere Ausführungsform der vorliegenden Erfindung ist daher dadurch gekennzeichnet, dass die Phase (II) des erfindungsgemäßen Mittels zusätzlich mindestens eine überwiegend öllösliche Pflegekomponente, ausgewählt aus öllöslichen Farbstoffen, öllöslichen Pflegestoffen, öllöslichen Vitaminen und Triglyceriden, enthält, wobei die zuvor genannten Mengen- und/oder Gewichtsverhältnisse der Phase (I) zur Phase (II) durch die Anwesenheit der zusätzlichen öllöslichen Pflegekomponente nicht überschritten werden.

Als überwiegend öllöslich werden erfindungsgemäß solche Verbindungen bezeichnet, die eine Wasserlöslichkeit von unter 1 g pro 1 L Wasser bei Normalbedingungen besitzen, jedoch in apolaren Verbindungen gut (d.h. > 10 g / kg Lösungsmedium) löslich sind.

Öllösliche Pflegestoffe sind beispielsweise kosmetisch wirksame Terpene und Terpenoide, wie beispielsweise Bisabolol, und Ubichinone, wie beispielsweise Coenzym Q-10.

Öllösliche Vitamine sind insbesondere die Verbindungen, die unter den Sammelbezeichnungen Vitamin A, Vitamin D, Vitamin E und Vitamin K bekannt sind. Ein erfindungsgemäß bevorzugtes Mittel enthält daher mindestens ein öllösliches Vitamin, ausgewählt aus Vitamin A, Vitamin D, Vitamin E und/oder Vitamin K sowie Vitamin P. Vitamin A umfasst dabei Retinoide, insbesondere *all-trans*-Retinol. Vitamin D, auch als Calciferole bezeichnet, umfasst 7,8-Didehydrosterol-Derivate, insbesondere die Verbindungen mit der Bezeichnung Cholecalciferol (Vitamin D₃, Calciol), Ergocalciferol (Vitamin D₂, Ercalciol), 7,8-Didehydrocholesterol (Provitamin D₃, Procalciol, Procholecalciferol) und Ergosterol (Provitamin D₂). Weitere, einsetzbare Vitamin D-Analoga sind Calcidiol (25-Hydroxycholecalciferol), Calcitriol, Hydroxycalcidiol und Vitamin D₁ (Ergocalciferol und Lumisterol). Vitamin E ist die Sammelbezeichnung für Tocopherole und umfasst insbesondere die chemischen Verbindungen α-Tocopherol, β-Tocopherol, γ-Tocopherol, δ-Tocopherol und α-Tocotrienol, β-Tocotrienol, γ-Tocotrienol und δ-Tocotrienol. Vitamin K ist Sammelbezeichnung für verschiedene Verbindungen mit Vitamin-K-Aktivität, die sich von 2-Methyl-1,4-naphthochinon (Vitamin K₃) ableiten. Bevorzugte Vertreter sind Vitamin K₁₍₂₀₎ (2-Methyl-3-phytyl-1,4-naphthochinon), Phyllochinon (Kurzzeichen: K),], Vitamin K₂(₃₅) (3-all-trans-Farnesylgeranylgeranyl-2-methyl-1,4-naphthochinon), Vitamin K₃ (2-Methyl-1,4-naphthochinon, Menadion, Menaphthon) sowie die abgeleiteten Analoga Vitamin K₄ (2-Methyl-1,4-naphthalindiol), Vitamin K₅ (4-Amino-2-methyl-1-naphthol), Vitamin K₆ (2-Methyl-1,4-naphthalindiamin) und Vitamin K₇ (4-Amino-3-methyl-1-naphthol). Bei Vitamin P handelt es sich um eine Sammelbezeichnung für Rutine, insbesondere Bioflavonoide wie Troxerutin (Vitamin P₄) und Hesperidin.

Triglyceride sind die Sammelbezeichnung für Ester des Glycerins, welche die Hauptbestandteile natürlicher Öle darstellen. Erfindungsgemäß besonders bevorzugte Triglyceride sind solche, die zumindest einen Ester einer ungesättigten Fettsäure enthalten. Bevorzugte ungesättigte Fettsäuren sind Ölsäure, Linolsäure und Linolensäure. Weiterhin können als Triglyceride bevorzugt pflanzliche Öle eingesetzt werden, insbesondere solche, die einen positiven Einfluss auf die Haaroberfläche besitzen. Besonders geeignete Triglyceride sind dabei insbesondere Öle, die aus den Saaten von Moringa pterygosperma (Moringaöl), aus den Kernen von Argania Spinosa (Arganöl) und/oder aus den Samen von Sclerocarya Birrea (Marulaöl) gewonnen werden.

Eine Ausführungsform des ersten Erfindungsgegenstands ist daher dadurch gekennzeichnet, dass die die hydrophobe Phase (II) zusätzlich mindestens ein Öl enthält, welches ausgewählt ist aus Ölen aus den Saaten von Moringa pterygosperma (Moringaöl), aus den Kernen von Argania Spinosa (Arganöl) und/oder aus den Samen von Sclerocarya Birrea (Marulaöl).

Bevorzugt werden die überwiegend öllöslichen Komponenten in einem Gesamtgewicht von 0,001 bis 5 Gew.-%, insbesondere von 0,01 bis 3 Gew.-%, jeweils bezogen auf das Gesamtgewicht der hydrophoben Phase (II), eingesetzt.

Die erfindungsgemäßen Mittel dienen bevorzugt der Farbveränderung keratinischer Fasern. Dazu wird das erfindungsgemäße, zweiphasige Mittel (M1) mit einem weiteren Mittel (M2), enthaltend mindestens eine farbverändernde Komponente, vermischt und die resultierende, anwendungsbereite Zubereitung auf die keratinischen Fasern gegeben.

Die erfindungsgemäßen Mittel (M1) weisen dabei, insbesondere zur Stabilisierung der Oxidationsmittel, bevorzugt einen sauren pH-Wert im Bereich von 2,5 bis 6 auf. Bei den pH-Werten im Sinne der vorliegenden Erfindung handelt es sich um pH-Werte, die bei einer Temperatur von 22 °C gemessen wurden.

Als farbverändernde Komponente in Mittel (M2) dienen als Aufhellmittel bevorzugt zusätzliche Bleichkraftverstärker, die die Wirkung des Oxidationsmittels aus Phase (I) des zweiphasigen erfindungsgemäßen Mittels verstärken, sowie farbgebende Komponenten.

In einer bevorzugten Ausführungsform enthält das Mittel (M2) daher einen zusätzlichen Bleichkraftverstärker. Als zusätzliche Bleichkraftverstärker können im Rahmen dieser Erfindung Peroxoverbindungen, weiterhin Verbindungen, die unter Perhydrolysebedingungen aliphatische Peroxocarbonsäuren und/oder substituierte Perbenzoesäure ergeben, Kohlensäurederivate, Alkylcarbonate, -carbamate, Silylcarbonate und -carbamate eingesetzt werden.

Vorzugsweise ist der Bleichkraftverstärker ausgewählt aus Ammoniumperoxodisulfat, Alkalimetallperoxodisulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Besonders bevorzugt sind Mittel, die als Bleichkraftverstärker mindestens ein anorganisches Salz, ausgewählt aus Peroxomonosulfaten und/oder Peroxodisulfaten, enthalten. Weiterhin hat es sich als besonders bevorzugt erwiesen, wenn die Mittel (M2) mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat. Die Peroxoverbindungen sind in einer Menge von 0,1 bis 25 Gew.-%, insbesondere in einer Menge von 0,5 bis 15 Gew.-%, bezogen auf das Gesamtgewicht des anwendungsbereiten Mittels, enthalten.

Der Einsatz von Persulfatsalzen bzw. Peroxodisulfatsalzen erfolgt in der Regel wasserfrei und in Form eines gegebenenfalls entstaubten Pulvers, Paste oder eines in Form gepressten Formkörpers. Die wasserfreien Mittel (M2) können anstelle und/oder zusätzlich zu den festen Peroxoverbindungen einen weiteren Bleichkraftverstärker enthalten.

Obwohl prinzipiell keine Einschränkungen hinsichtlich der Formulierung der weiteren Mittel (M2) bestehen, kann es erfindungsgemäß bevorzugt sein, wenn Mittel (M2) wasserfrei formuliert werden. Wasserfrei im Sinne der vorliegenden Erfindung bedeutet einen Wassergehalt - bezogen auf die Mittel (M2) - von weniger als 5 Gew.-%, insbesondere von weniger als 2 Gew.-%. Blondierzubereitungen, die weniger als 0,1 Gew.-% Wasser enthalten, können bevorzugt sein.

In einer weiteren, bevorzugten Ausführungsform kann das Mittel (M2) als Bleichkraftverstärker mindestens ein kationisches Pyridinium-Derivat enthalten. Bevorzugte Verbindungen sind 4-Acyl-Pyridinium-Derivate und 2-Acylpyridinium-Derivate. Insbesondere bevorzugt sind dabei 2-Acetyl-1-methylpyridinium-p-toluolsulfonat und 4-Acetyl-1-methylpyridinium-p-toluolsulfonat. Weiterhin bevorzugte kationische Pyridinium-Derivate sind dabei kationische 3,4-Dihydroisochinolinium-Derivat. Insbesondere bevorzugt ist N-Methyl-3,4-dihydroisochinolinium-p-toluolsulfonat.

Die neben oder anstelle von Peroxoverbindungen eingesetzten Bleichkraftverstärker können in den Mitteln (M2) bevorzugt in Mengen von 0,05 bis 10 Gew.-%, insbesondere in Mengen von 0,2 bis 5 Gew.-%, jeweils bezogen auf das Gesamtgewicht des Mittels (M2), enthalten sein.

In einer weiteren bevorzugten Ausführungsform enthält das Mittel (M2) als farbverändernde Komponente farbgebende Komponenten. Diese können bevorzugt ausgewählt sein aus mindestens einem Oxidationsfarbstoffvorprodukt und/oder aus mindestens einem direktziehenden Farbstoff.

Erfindungsgemäß bevorzugte Mittel zur Farbveränderung keratinischer Fasern sind dadurch gekennzeichnet, dass sie mindestens ein Oxidationsfarbstoffvorprodukt enthalten. Insbesondere bevorzugt sind Mittel, die mindestens ein Oxidationsfarbstoffvorprodukt vom Entwicklertyp (Entwicklerkomponente), bevorzugt in Kombination mit mindestens einem Oxidationsfarbstoffvorprodukt vom Kupplertyp (Kupplerkomponente) enthalten.

Bevorzugte Oxidationsfarbstoffvorprodukte vom Entwicklertyp sind p-Phenylendiaminderivate. Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetyl-aminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie deren physiologisch verträglichen Salzen. Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines ihrer physiologisch verträglichen Salze. Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Ganz besonders bevorzugte Verbindungen sind p-Aminophenol, 4-Amino-3-methylphenol, 4-Amino-2-aminomethyl-phenol, 4-Amino-2-(1,2-dihydroxyethyl)phenol und 4-Amino-2-(diethylaminomethyl)phenol. Ferner kann die Entwicklerkomponente ausgewählt sein aus o-Aminophenol und seinen Derivaten, wie 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie Pyrimidinderivaten, Pyrazolderivaten, Pyrazolopyrimidin-und Pyrazolopyrazol-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind insbesondere die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Bevorzugte Pyrazolopyrimidine sind die Verbindungen, die ausgewählt werden unter Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)-amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen, wenn ein tautomeres Gleichgewicht vorhanden ist. Bevorzugtes Pyrazolopyrazol-Derivat ist 2,3-Diamino-6,7-dihydro-1H,5H-pyrazolo[1,2-a]pyrazol-1-on.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diaza-cycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen. Ganz besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)-pyrazol sowie deren physiologisch verträglichen Salze.

Die Entwicklerkomponenten werden bevorzugt in einer Menge von 0,0001 bis 0,5 Gew.-%, vorzugsweise 0,001 bis 0,2 Gew.-%, jeweils bezogen auf das anwendungsbereite Mittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt. Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus.

Erfindungsgemäße Kupplerkomponenten werden bevorzugt als mindestens eine Verbindung aus einer der folgenden Klassen ausgewählt: m-Aminophenol, o-Aminophenol, m-Diaminobenzol, o-Diaminobenzol und/oder deren Derivate; Naphthalinderivate mit mindestens einer Hydroxygruppe; Di- beziehungsweise Trihydroxybenzol; Pyridinderivate; Pyrimidinderivate; bestimmte Indol-Derivate und Indolin-Derivate; Pyrazolonderivate (beispielsweise 1-Phenyl-3-methylpyrazol-5-on); Morpholinderivate (beispielsweise 6-Hydroxybenzomorpholin oder 6-Aminobenzomorpholin); Chinoxalinderivate (beispielsweise 6-Methyl-1,2,3,4-tetrahydrochinoxalin), sowie Gemische aus zwei oder mehreren Verbindungen aus einer oder mehreren dieser Klassen.

Bevorzugte m-Aminophenol-Kupplerkomponenten werden ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen. Bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diamino-phenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen. Bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und deren physiologisch verträglichen Salzen. Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methyl-amino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und deren physiologisch verträglichen Salzen. Bevorzugte Pyrimidinderivate werden ausgewählt aus mindestens einer Verbindung der Gruppe, die gebildet wird aus 4,6-Diaminopyrimidin, 4-Amino-2,6-di-hydroxypyrimidin, 2,4-Diamino-6-hydroxypyrimidin, 2,4,6-Trihydroxypyrimidin, 2-Amino-4-methyl-pyrimidin, 2-Amino-4-hydroxy-6-methylpyrimidin und 4,6-Dihydroxy-2-methylpyrimidin und deren physiologisch verträglichen Salzen. Bevorzugte Indolderivate werden ausgewählt aus mindestens einer Verbindung der Gruppe, die gebildet wird aus 4-Hydroxyindol, 6-Hydroxyindol und 7-Hydroxyindol und deren physiologisch verträglichen Salzen. Bevorzugte Indolinderivate werden ausgewählt aus mindestens einer Verbindung der Gruppe, die gebildet wird aus 4-Hydroxyindolin, 6-Hydroxyindolin und 7-Hydroxyindolin und deren physiologisch verträglichen Salzen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-di-aminophenoxy)propan, 1-Methoxy-2-amino-4-(2-hydroxyethylamino)benzol, 1,3-Bis(2,4-diamino-phenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Di-amino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Ganz besonders bevorzugt sind Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 0,5 Gew.-%, vorzugsweise 0,001 bis 0,2 Gew.-%, jeweils bezogen auf das anwendungsbereite Mittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Weiterhin können die farbverändernden Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe können in anionische, kationische und nichtionische direktziehende Farbstoffe unterteilt werden. Üblicherweise sind es Nitrophenylendiamine, Nitroaminophenole, Azofarbstoffe, Anthrachinone oder Indophenole. Die direktziehenden Farbstoffe werden jeweils bevorzugt in einer Menge von 0,0001 bis 0,2 Gew.-%, bevorzugt von 0,001 bis 0,1 Gew.-%, jeweils bezogen auf die gesamte Anwendungszubereitung, eingesetzt. Die Gesamtmenge an direktziehenden Farbstoffen beträgt vorzugsweise höchstens 0,1 Gew.-%.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor® vertrieben werden, sind erfindungsgemäß ebenfalls besonders bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)-amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Erfindungsgemäß bevorzugte Farbstoffkombinationen sind solche, die mindestens die Kombination aus Tetrabromphenolblau und Acid Red 92; Tetrabromphenolblau und Acid Red 98; Tetrabromphenolblau und Acid Red 94; Tetrabromphenolblau und Acid Red 87; oder Tetrabromphenolblau und Acid Red 51 enthalten.

Erfindungsgemäße, anwendungsbereite Mittel sind vorzugsweise wässrige, fließfähige Zubereitungen. Die erfindungsgemäßen Mittel können weiterhin alle für solche Zubereitungen bekannten Wirk-, Zusatz- und Hilfsstoffe enthalten.

Die Oxidationsmittelzubereitung kann auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, lodide, Chinone oder Metallionen. Hierfür geeignete Enzyme sind z. B. Peroxidasen, die die Wirkung geringer Mengen an Wasserstoffperoxid deutlich verstärken können. Ein Einsatz bestimmter Metallionen oder -komplexe kann ebenfalls bevorzugt sein. Geeignete Metallionen sind beispielsweise Zn²⁺, Cu²⁺, Fe²⁺, Fe³⁺, Mn²⁺, Mn⁴⁺, Li⁺, Mg²⁺, Ca²⁺, Ce⁴⁺, V³⁺, Co²⁺, Ru³⁺ und Al³⁺. Besonders geeignet sind dabei Zn²⁺, Cu²⁺ und Mn²⁺.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind so genannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metallatomen in der Regel unter Bildung von Chelat-Komplexen reagieren, sind erfindungsgemäß einsetzbar. Die Polymer-gebundenen Liganden der entstehenden Metall-Komplexe können dabei aus nur einem Makromolekül stammen oder aber zu verschiedenen Polymerketten gehören. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/ oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Weitere, erfindungsgemäß einsetzbare Wirk-, Hilfs- und Zusatzstoffe sind beispielsweise nichtionische Polymere (wie Vinylpyrrolidinon/Vinylacrylat-Copolymere, Polyvinylpyrrolidinon und Vinylpyrrolidinon/Vinylacetat-Copolymere und Polysiloxane); zwitterionische und amphotere Polymere (wie Acrylamidopropyltrimethylammoniumchlorid/Acrylat-Copolymere und Octylacrylamid/Methyl-methacrylat/tert-Butylaminoethylmethacrylat/2-Hydroxypropylmethacrylat-Copolymere); Verdickungsmittel (wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie Bentonit oder vollsynthetische Hydrokolloide wie z.B. Polyvinylalkohol); Strukturanden (wie Zucker, Maleinsäure und Milchsäure) und Konsistenzgeber (wie Zuckerester, Polyolester oder Polyolalkylether); Proteinhydrolysate (insbesondere Elastin-, Kollagen-, Keratin-, Milcheiweiß-, Sojaprotein- und Weizenproteinhydrolysate, deren Kondensationsprodukte mit Fettsäuren); Parfümöle; Cyclodextrine; Lösungsmittel und -vermittler (wie Ethanol, Isopropanol, Ethylenglykol, Propylenglykol, Glycerin, Dimethylisosorbid und Diethylenglykol); Entschäumer wie Silicone; Farbstoffe und Pigmente zum Anfärben des Mittels; Antischuppenwirkstoffe (wie Piroctone Olamine, Zink Omadine und Climbazol); Lichtschutzmittel (insbesondere derivatisierte Benzophenone, Zimtsäure-Derivate und Triazine); Wirkstoffe (wie Allantoin, Pyrrolidoncarbonsäuren, Cholesterin und deren Salze); weitere Fette und Wachse (wie Fettalkohole, Bienenwachs, Montanwachs und Paraffine); Quell- und Penetrationsstoffe (wie Glycerin, Propylenglykolmonoethylether, Carbonate, Hydrogencarbonate, Guanidine, Harnstoffe sowie primäre, sekundäre und tertiäre Phosphate); Trübungsmittel (wie Latex, Styrol/PVP- und Styrol/Acrylamid-Copolymere); Perlglanzmittel (wie Ethylenglykolmono- und -distearat sowie PEG-3-distearat); Treibmittel (wie Propan-Butan-Gemische, N₂O, Dimethylether, CO₂ und Luft) sowie Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Bevorzugte gebrauchsfertige Mittel, bestehend aus erfindungsgemäßem zweiphasigem Mittel (M1) und Farbveränderungsmittel (M2), weisen bevorzugt einen pH-Wert im Bereich von 6 bis 12 auf. Besonders bevorzugte Mittel sind dadurch gekennzeichnet, dass sie einen alkalischen pH-Wert besitzen. Eine weitere bevorzugte Ausführungsform der vorliegenden Erfindung besteht darin, dass das gebrauchsfertige Mittel einen pH-Wert zwischen 7,0 und 12,0, bevorzugt zwischen 8,0 und 11,0 besitzt. Bei den pH-Werten im Sinne der vorliegenden Erfindung handelt es sich um pH-Werte, die bei einer Temperatur von 22 °C gemessen wurden.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Erfindungsgemäß ganz besonders bevorzugte Alkanolamine werden ausgewählt aus der Gruppe 2-Aminoethan-1-ol (Monoethanolamin), 2-Amino-2-methylpropan-1-ol und 2-Amino-2-methyl-propan-1,3-diol. Ein insbesondere bevorzugtes Alkanolamin ist Monoethanolamin. Geeignete basische Aminsäuren sind Lysin, Arginin und Ornithin. Das erfindungsgemäße, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat.

Die Anwendungstemperaturen können in einem Bereich zwischen 15 und 40 °C liegen. Nach einer Einwirkungszeit von 2 bis 60, bevorzugt 5 bis 45 Minuten wird das Blondiermittel durch Ausspülen von dem Haar entfernt. Das Nachwaschen mit einem Shampoo entfällt, wenn ein stark tensidhaltiger Träger verwendet wurde.

Wichtig ist, dass die Mittel (M1) und (M2) hinreichend flüssig sind, damit eine gute Vermischung untereinander möglich ist. Dazu ist vorteilhaft, wenn beide Mittel nicht als Paste, zähe Creme oder eingedicktes Gel vorliegen, sondern über eine ausreichende Fließfähigkeit verfügen. Weiterhin müssen die anwendungsbereiten Mittel nach der Vermischung der Einzelkomponenten zwar über rheologische Eigenschaften verfügen, die ein Auftragen auf die zu färbenden Fasern erlauben, aber gleichzeitig ein Verlaufen oder Ausfliessen des Mittels vom Wirkort während Anwendungsdauer verhindern. Bevorzugt besitzen die Anwendungsmischungen daher eine Viskosität von 5 bis 100 Pa·s, bevorzugt von 10 bis 50 Pa·s , insbesondere von 10 bis 20 Pa·s und insbesondere bevorzugt von 10 bis 16 Pa·s (Brookfield, 22°C, Spindel #5, 4 rpm).

Je nach Zusammensetzung der Mittel (M1) und (M2) ist es bevorzugt, die Anwendungsmischungen erst unmittelbar vor der Anwendung durch Vermischen von Mittel (M1) und Mittel (M2) herzustellen. Dies kann insbesondere bei Inkompatibilitäten zwischen einzelnen Inhaltsstoffen vorteilhaft sein. Daher ist eine bevorzugte Darreichungsform des anwendungsbereiten Mittels eine getrennte Verpackungseinheit, worin die Mittel (M1) und (M2) jeweils getrennt voneinander verpackt vorliegen.

Ein weiterer Gegenstand der vorliegenden Erfindung ist daher eine Mehrkomponentenverpackungseinheit (Kit-of-Parts), welche mindestens zwei voneinander getrennt konfektionierte Container enthält, wobei ein erster Container (C1) ein kosmetisches Mittel (M1) gemäß dem ersten Erfindungsgegenstand enthält, und ein zweiter Container (C2) eine Farbveränderungszubereitung (M2) enthält, wobei die Farbveränderungszubereitung (M2) in einem kosmetischen Träger mindestens eine farbverändernde Komponente, bevorzugt mindestens ein Oxidationsfarbstoffvorprodukt, enthält.

Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff. Besonders bevorzugt zur Visualisierung der zweiphasigen Mittel (M1) ist eine Ausführungsform, bei der die Umhüllung des Containers, welcher das Mittel (M1) enthält, für den Anwender durchsichtig ist. Eine bevorzugte Ausführungsform der erfindungsgemäßen Mehrkomponentenverpackungseinheit ist daher dadurch gekennzeichnet, dass der erste Container (C1), enthaltend das Mittel (M1), eine durchsichtige Verpackung, bevorzugt eine durchsichtige Kunststoffverpackung, besitzt.

Weiterhin kann es erfindungsgemäß besonders vorteilhaft sein, wenn das genannte Kit-of-Parts mindestens ein weiteres Haarbehandlungsmittel in einem getrennten Container enthält, insbesondere ein Konditioniermittel. Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen.

Hinsichtlich der bevorzugten Ausführungsführungsformen der Mittel (M1) und (M2) gelten mutatis mutandis die obigen Ausführungen der vorangehenden Erfindungsgegenstände.

Bei der Anwendung der Mehrkomponentenverpackungseinheit kann es unerheblich sein, ob zunächst die beiden Phasen von Mittel (M1) durch kräftiges Schütteln kurzzeitig durchmischt werden und vor erneuter Phasentrennung mit dem Mittel (M2) vereinigt wird, um die anwendungsbereite Farbveränderungszubereitung bereitzustellen, oder ob zunächst das Mittel (M2) mit Mittel (M1) vereinigt wird und anschließend durch inniges Vermischen die anwendungsbereite Mischung hergestellt wird.

Ein weiterer Erfindungsgegenstand ist daher ein Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand die beiden Mittel (M1) und (M2) in einem der Container (C2) oder (C1) vereinigt werden, der wiederverschlossene Container daraufhin geschüttelt wird, und das im Container resultierende, anwendungsbereite Farbveränderungsmittel anschließend auf die Fasern aufgebracht wird, für eine Einwirkdauer von 5 bis 60 Minuten auf den Fasern belassen und schließlich ausgespült wird.

Zur verbesserten Durchmischung ist vorteilhaft, wenn der Container (C1), welcher das zweiphasige Mittel (M1) enthält, eine wieder-verschließbare Öffnung, wie beispielsweise einen Schnapp- oder einen Schraubverschluss, besitzt. Dies ermöglicht die erleichterte Zugabe des farbverändernden Mittels aus Container (C2), welcher seinerseits bevorzugt in Form eines Tütchens oder Sachets im Falle von wasserfreien, insbesondere pulverförmigen Farbveränderungsmitteln, oder in Form einer Tube im Falle von fließfähigen Farbveränderungsmitteln. Es ist bevorzugt, die einzelnen Zubereitungen zu vermischen und das anwendungsbereite Mittel zeitnah auf die keratinischen Fasern zu applizieren.

Eine besonders bevorzugte Ausführungsform dieses Erfindungsgegenstands ist daher ein Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand der Inhalt des Containers (C2) in den Container (C1) gefüllt wird, der wiederverschlossene Container (C1) daraufhin geschüttelt wird, und das im Container (C1) resultierende, anwendungsbereite Farbveränderungsmittel anschließend auf die Fasern aufgebracht wird, für eine Einwirkdauer von 5 bis 60 min auf den Fasern belassen und dann ausgespült wird.

Ein weiterer Erfindungsgegenstand ist schließlich ein Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand der Container (C1) geschüttelt wird, die entstandene Mischung der Phasen (I) und (II) unmittelbar anschließend mit einer Färbezubereitung des Containers (C2) innig vermischt, das resultierende, anwendungsbereite Farbveränderungsmittel anschließend auf die Fasern aufgebracht wird, für eine Einwirkdauer von 5 bis 60 min auf den Fasern belassen und schließlich ausgespült wird.

Im Falle eines farbgebenden Mittels beträgt die bevorzugte Einwirkzeit 5 bis 40 Minuten, bevorzugt 10 bis 30 Minuten. Im Falle von aufhellenden oder bleichenden Farbveränderungsmitteln liegt die bevorzugte Einwirkzeit bei 30 bis 60 Minuten, bevorzugt bei 40 bis 60 Minuten.

Im Rahmen dieses Gegenstandes der Erfindung gelten mutatis mutandis die oben getroffenen Aussagen analog. Die folgenden Beispiele sollen den Gegenstand der vorliegenden Erfindung näher erläutern ohne diesen in irgendeiner Weise zu beschränken.

### Beispiele

### 1) Zweiphasige Entwicklerzubereitunqen (Tabelle 1; Mengenangaben in Gew.-%)

Folgende zweiphasige Entwicklerzubereitungen (Zubereitung 1 stellt eine Referenzzubereitung dar) wurden mit unterschiedlichen hydrophoben Ölmischungen hergestellt.

**Tabelle 1**

| **Rohstoffe:** | **1** | **2** | **3** |
|---|---|---|---|
| **Wässrige Phase:** | | | |
| Natronlauge, 45 % techn. | 0,76 | 0,76 | 0,76 |
| Dipicolinsäure | 0,10 | 0,10 | 0,10 |
| Dinatriumpyrophosphat | 0,03 | 0,03 | 0,03 |
| HEDP, wässrig, 60%ig | 1,35 | 1,35 | 1,35 |
| Natriumlaurethsulfat (2EO), 27%iq | 1,80 | 1,80 | 1,80 |
| Aculyn® 33A | 15,50 | 15,50 | 15,50 |
| Wasserstoffperoxid, wässrig, 50%ig | 12,00 | 12,00 | 12,00 |
| | | | |

| **Hydrophobe Ölphase:** | | | |
|---|---|---|---|
| Isopropylpalmitat | 4,60 | 4,60 | 4,60 |
| Controx® KS C | 0,07 | 0,07 | 0,07 |
| Isopropylisostearat | 4,90 | -- | -- |
| Eutanol® G16S | -- | 4,90 | -- |
| 2-Ethylhexylpalmitat | -- | -- | 4,90 |
| Marulaöl | 0,10 | 0,10 | 0,10 |
| Wasser, vollentsalzt | ad 100 | | |

Rohstoffe: Aculyn 33A (ca. 28%; INCI-Bezeichnung: Acrylates Copolymer, Aqua; Rohm & Haas); Controx KS C (INCI-Bezeichnung: Tocopherol, Hydrogenated Palm Glycerides Citrate; BASF); Eutanol G16S (INCI-Bezeichnung: Hexyldecyl Stearate; BASF).

Zur Herstellung der Entwicklerzubereitungen wurden die Rohstoffe mit Ausnahme der Öle (Isopropylpalmitat, Controx KS C, Isopropylisostearat, Marulaöl (1); Isopropylpalmitat, Controx KS C, Eutanol G16S, Marulaöl (2) und Isopropylpalmitat, Controx KS C, 2-Ethylhexylpalmitat, Marulaöl (3)) vorgemischt. Anschließend wurde die jeweilige hydrophobe, klare Ölphase zugegeben.

Die wässrige Phase und die hydrophobe Ölphase liegen im Ruhezustand getrennt voneinander vor. Sie lassen sich durch Schütteln hervorragend vermischen. Die Lagerung des zweiphasigen Mittels bereitete keinerlei Probleme. Auch bei Temperaturschwankungen und der Lagerung bei kälteren Temperaturen (< 15°C) konnten keine Veränderungen festgestellt werden.

### 2) Färbecremes I-III (Tabelle 2; Mengenangaben in Gew.-%)

| **Rohstoffe** | **I** | **II** | **III** |
|---|---|---|---|
| Lanette® D | 6,60 | 6,60 | 6,60 |
| Lorol® C12-18 techn. | 2,40 | 2,40 | 2,40 |
| Eumulgin® B 2 | 0,60 | 0,60 | 0,60 |
| Eumulgin® B 1 | 0,60 | 0,60 | 0,60 |
| Lamesoft® PO 65 | 2,00 | 2,00 | 2,00 |
| Akypo Soft® 45HP | 10,00 | 10,00 | 10,00 |
| Texapon® K 14 S Special, 70 % | 2,80 | 2,80 | 2,80 |
| Produkt W 37194® | 3,75 | 3,75 | 3,75 |
| p-Toluylendiaminsulfat | 0,37 | 2,21 | 1,53 |
| 2,4,5,6-Tetraaminopyrimidinsulfat | 1,46 | | |
| Resorcin | 0,10 | 0,79 | 0,41 |
| 2-Methylresorcin | 0,90 | | 0,34 |
| m-Aminophenol | | 0,29 | |
| Lehmanns Blau | | 0,05 | |
| 2-Amino-3-hydroxypyridin | | | 0,05 |
| Ammoniumsulfat | 0,02 | | |
| Natriumsulfit, wasserfrei | 0,40 | 0,40 | 0,40 |
| Ascorbinsäure | 0,10 | 0,10 | |
| Natriumhydroxid, wässrig, 45% | 1,90 | 2,00 | 1,50 |
| Natriumsilikat 40/42 | 0,50 | 0,50 | 0,50 |
| L-Serin | 1,00 | 1,00 | 1,00 |
| Ajidew® NL 50 | | | 1,00 |
| Turpinal® SL | 0,20 | 0,20 | 0,20 |
| Parfum | 0,30 | 0,30 | 0,30 |
| Wasser, vollentsalzt | ad 100 | ad 100 | ad 100 |

Rohstoffe: Lanette D (INCI-Bezeichnung: Cetearyl alcohol; BASF); Lorol C12-18 techn. (INCI-Bezeichnung: Coconut alcohol; BASF); Eumulgin B 2 (INCI-Bezeichnung: Ceteareth-20; BASF); Eumulgin B 1 (INCI-Bezeichnung: Ceteareth-12; BASF); Akypo Soft 45HP (ca. 21 %, INCI-Bezeichnung: Sodium Laureth-6 Carboxylate, Aqua; KAO); Texapon K 14 S Special (ca. 70 %, INCI-Bezeichnung: Sodium Myreth Sulfate, Aqua; BASF); Produkt W 37194 (ca. 20 %, INCI-Bezeichnung: Acrylamidopropyltrimonium Chloride / Acrylates Copolymer, Aqua; Stockhausen);

Ajidew® NL 50 (INCI-Bezeichnung: Sodium PCA; Ajinomoto); Turpinal® SL (INCI-Bezeichnung: Etidronic acid, aqua; Thermphos).

Die Fettbasis wurde jeweils bei 80 °C aufgeschmolzen und mit einem Teil der Wassermenge dispergiert. Anschließend wurden die restlichen Rezepturbestandteile unter Rühren der Reihe nach eingearbeitet. Es wurde mit Wasser auf 100 Gew.-% aufgefüllt und die Formulierungen I-III kalt gerührt.

Die jeweilige Färbecreme wurde vor der Anwendung mit einer der Entwicklerlösungen bei Raumtemperatur im Gewichtsverhältnis von 1:1 versetzt und innig vermischt.

## Patentansprüche

1. Kosmetisches Mittel für die Behandlung keratinischer Fasern, **dadurch gekennzeichnet, dass** es mindestens zwei nebeneinander vorliegende, aber durch eine Phasengrenze voneinander getrennte Phasen umfasst,
wobei die erste Phase (I) eine wässrige Phase darstellt, die mindestens ein chemisches Oxidationsmittel enthält, und
wobei die zweite Phase (II) eine hydrophobe Ölphase darstellt, die eine Mischung aus
- Isopropylpalmitat (II-1) und
- mindestens einem weiteren flüssigen, verzweigten Carbonsäureester (II-2), ausgewählt aus 2-Ethylhexylpalmitat, Hexyldecylpalmitat, Hexyldecylstearat, Hexyldecyllaurat, Isodecylneopentanoat, Isononylisononanoat, 2-Ethylhexylstearat, Isooctylstearat, Isononylstearat, Isocetylstearat, Isotridecylisononanoat, Propylheptylcaprylat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat und/oder Diisotridecylacetat, enthält.

2. Mittel nach Anspruch 1, **dadurch gekennzeichnet, dass** das chemische Oxidationsmittel der Phase (I) ausgewählt ist aus Wasserstoffperoxid und/oder einem seiner festen Anlagerungsprodukte an anorganische und/oder organische Verbindungen.

3. Mittel nach einem der Ansprüche 1 oder 2, **dadurch gekennzeichnet, dass** das Gewichtsverhältnis des Isopropylpalmitats (II-1) zu der Summe aller weiterer Carbonsäureester (II-2) in der Phase (II) 20 : 80 bis 80 : 20, bevorzugt 30 : 70 bis 70 : 30, mehr bevorzugt 40 : 60 bis 60 : 40 und insbesondere bevorzugt 45 : 55 bis 55 : 45 beträgt.

4. Mittel nach einem der Ansprüche 1 bis 3, **dadurch gekennzeichnet, dass** es - bezogen auf sein Gesamtgewicht - 3 bis 30 Gew.-%, bevorzugt 4 bis 25 Gew.-%, besonders bevorzugt 5 bis 20 Gew.-% und insbesondere 7,5 bis 15 Gew.-% hydrophobe Ölphase (II) enthält.

5. Mittel nach einem der Ansprüche 1 bis 4, **dadurch gekennzeichnet, dass** die wässrige Phase (I) weiterhin mindestens ein anionisches, polymeres Verdickungsmittel enthält, das ausgewählt ist aus vernetzten oder unvernetzten Copolymeren, die mindestens zwei unterschiedliche Monomere aus der Gruppe der Acrylsäure, Methacrylsäure, den C₁-C₆-Alkylestern der Acrylsäure und/oder den C₁-C₆-Alkylestern der Methacrylsäure enthalten.

6. Mittel nach einem der Ansprüche 1 bis 5, **dadurch gekennzeichnet, dass** das Mittel nichtionische, anionische, zwitterionische und/oder amphotere Tenside und/oder Emulgatoren in einem Gesamtgewicht von weniger als 5 Gew.-%, bevorzugt von weniger als 3 Gew.-%, jeweils bezogen auf das Gesamtgewicht des Mittels, enthält.

7. Mehrkomponentenverpackungseinheit (Kit-of-Parts), enthaltend mindestens zwei voneinander getrennt konfektionierte Container, wobei ein erster Container (C1) eine kosmetische Zubereitung (M1) gemäß einem der Ansprüche 1 bis 6 und ein zweiter Container (C2) eine Farbveränderüngszubereitung (M2) enthält, wobei die Farbveränderungszubereitung (M2) in einem kosmetischen Träger mindestens eine farbverändernde Komponente, bevorzugt mindestens ein Oxidationsfarbstoffvorprodukt, enthält.

8. Verfahren zur Farbveränderung keratinischer Fasern, insbesondere menschlicher Haare, **dadurch gekennzeichnet, dass** aus einer Mehrkomponentenverpackungseinheit gemäß Anspruch 7 die beiden Mittel (M1) und (M2) in einem der Container (C2) oder (C1) vereinigt werden, der wiederverschlossene Container daraufhin geschüttelt wird, und das im Container resultierende, anwendungsbereite Farbveränderungsmittel anschließend auf die Fasern aufgebracht wird, für eine Einwirkdauer von 5 bis 60 min auf den Fasern belassen und schließlich ausgespült wird.

## Claims

1. A cosmetic agent for treating keratinic fibers, **characterized in that** the cosmetic agent comprises at least two phases that are adjacent to one another but are separated from one another by a phase boundary,
the first phase (I) being an aqueous phase that contains at least one chemical oxidizing agent, and
the second phase (II) being a hydrophobic oil phase that contains a mixture of
- isopropyl palmitate (11-1) and
- at least one additional liquid, branched carboxylic acid ester (II-2), selected from 2-ethylhexyl palmitate, hexyldecyl palmitate, hexyldecyl stearate, hexyldecyl laurate, isodecyl neopentanoate, isononyl isononanoate, 2-ethylhexyl stearate, isooctyl stearate, isononyl stearate, isocetyl stearate, isotridecyl isononanoate, propylheptyl caprylate, cetearyl isononanoate, 2-ethylhexyl laurate, 2-ethylhexyl isostearate, 2-ethylhexyl cocoate, 2-octyldodecyl palmitate, butyloctanoic acid 2-butyl octanoate and/or diisotridecyl acetate.

2. The agent according to claim 1, **characterized in that** the chemical oxidizing agent of phase (I) is selected from hydrogen peroxide and/or one of the solid addition products thereof with inorganic and/or organic compounds.

3. The agent according to one of claims 1 or 2, **characterized in that** the weight ratio of the isopropyl palmitate (11-1) to the sum of all of the other carboxylic acid esters (II-2) in phase (II) is from 20:80 to 80:20, preferably from 30:70 to 70:30, more preferably from 40:60 to 60:40 and particularly preferably from 45:55 to 55:45.

4. The agent according to one of claims 1 to 3, **characterized in that** the agent contains, based on its total weight, from 3 to 30 wt.%, preferably from 4 to 25 wt.%, particularly preferably from 5 to 20 wt.% and in particular from 7.5 to 15 wt.% hydrophobic oil phase (II).

5. The agent according to one of claims 1 to 4, **characterized in that** the aqueous phase (I) further contains at least one anionic, polymeric thickening agent that is selected from crosslinked or non-crosslinked copolymers that contain at least two different monomers from the group of acrylic acid, methacrylic acid, the C₁-C₆ alkyl esters of acrylic acid and/or the C₁-C₆ alkyl esters of methacrylic acid.

6. The agent according to one of claims 1 to 5, **characterized in that** the agent contains nonionic, anionic, zwitterionic and/or amphoteric surfactants and/or emulsifiers in a total weight of less than 5 wt.%, preferably of less than 3 wt.%, based in each case on the total weight of the agent.

7. A multicomponent packaging unit (kit of parts), containing at least two separately packaged containers, wherein a first container (C1) contains a cosmetic preparation (M1) according to one of claims 1 to 6 and a second container (C2) contains a color-changing preparation (M2), wherein the color-changing preparation (M2) contains, in a cosmetic carrier, at least one color-changing component, preferably at least one oxidation dye precursor.

8. A method for modifying the color of keratinic fibers, in particular human hair, **characterized in that**, from a multicomponent packaging unit according to claim 7, the two agents (M1) and (M2) are combined in one of the containers (C2) or (C1), the re-closed container is then shaken, and the ready-to-apply color-changing agent that results in the container is then applied to the fibers, left on the fibers for a contact time of from 5 to 60 minutes, and finally rinsed out.

## Revendications

1. Produit cosmétique pour le traitement de fibres de kératine, **caractérisé en ce qu'**il contient au moins deux phases contiguës mais séparées par une limite de phase, la première phase (I) présentant une phase aqueuse qui contient au moins un oxydant chimique, et la deuxième phase (II) présentant une organique hydrophobe contenant un mélange de :
- palmitate d'isopropyle (II-1), et
- au moins un autre ester d'acide carboxylique liquide (II-2) choisi parmi le palmitate de 2-éthylhexyle, palmitate d'hexyldécyle, stéarate d'hexyldécyle, laurate d'hexyldécyle, néopentanoate d'isodécyle, isononanoate d'isononyle, stéarate de 2-éthylhexyle, stéarate d'isooctyle, stéarate d'isononyle, stéarate d'isocétyle, isononanoate d'isotridécyle, caprylate de propylheptyle, isononanoate de cétéaryle, laurate de 2-éthylhexyle, isostéarate de 2-éthylhexyle, cocoate de 2-éthylhexyle, palmitate de 2-octyldodécyle, butyloctanoate de 2-butyloctyle et/ou acétate de diisotridécyle.

2. Produit selon la revendication 1, **caractérisé en ce que** l'oxydant de la phase (I) est choisi parmi le peroxyde d'hydrogène et/ou un de ses produits d'addition avec des composés organiques ou minéraux.

3. Produit selon la revendication 1 ou 2, **caractérisé en ce que** le rapport pondéral du palmitate d'isopropyle (II-1) à la somme de tous les autres esters d'acide carboxylique (II-2) dans la phase (II) est 20:80 à 80:20, de préférence 30:70 à 70:30, plus préférentiellement 40:60 à 60:40 et en particulier 45:55 à 55:45.

4. Produit selon une des revendications 1 à 3, **caractérisé en ce qu'**il contient 3 à 30 % en poids, de préférence 4 à 25 % en poids, particulièrement préférentiellement 5 à 20 % en poids et en particulier 7,5 à 15 % en poids de phase organique hydrophobe (II) rapporté au poids total.

5. Produit selon une des revendications 1 à 4, **caractérisé en ce que** la phase aqueuse (I) contient en outre au moins un épaississant polymère anionique choisi parmi des copolymères réticulés ou non-réticulés contenant au moins deux monomères différents issus du groupe de l'acide acrylique, acide méthacrylique, esters de C₁₋₆-alkyle de l'acide acrylique et/ou C₁₋₆-alkyle de l'acide méthacrylique.

6. Produit selon une des revendications 1 à 5, **caractérisé en ce que** le produit contient des tensioactifs et/ou émulsifiants non-ioniques, zwitterioniques et/ou amphotères à hauteur d'un poids total inférieur à 5 % en poids, préférentiellement inférieur à 3 % en poids, respectivement rapporté au poids total du produit.

7. Unité de conditionnement multicomposant (kit) contenant au moins deux récipients confectionnés séparés l'un de l'autre, le premier récipient (C1) contenant une préparation cosmétique (M1) selon une des revendications 1 à 6 et un deuxième récipient (C2) contenant une préparation de modification de couleur (M2), la préparation de modification de couleur (M2) contenant dans un vecteur cosmétique un composant modificateur de couleur, de préférence au moins un précurseur de coloration oxydante.

8. Préparation de modification de la couleur de fibres de kératine, en particulier de cheveux humains, **caractérisée en ce qu'**à partir d'une unité de conditionnement multicomposant selon la revendication 7, les deux produits (M1) et (M2) sont réunis dans le récipient (C2) ou (C1), le récipient refermé est ensuite agité, et la préparation de modification de couleur qui en résulte dans le récipient est ensuite appliquée sur les fibres, laissée à agir pour une durée de 5 à 60 min sur les fibres, et enfin rincée.
